# EUROPÄISCHE PATENTANMELDUNG

(11) **EP 1 016 415 A2**
(43) Veröffentlichungstag der Anmeldung: **05.07.2000**
(21) Anmeldenummer: 99124688.5
(22) Anmeldetag: 11.12.1999
(51) Int. Cl.: A61K 38/36, A61K 35/16, C07K 14/745

(54) **Verwendung eines Fibrinklebers zur Geweberegeneration**

(30) Priorität: 18.12.1998 DE 19858463
(71) Anmelder: Centeon Pharma GmbH, 35002 Marburg (DE)
(72) Erfinder: Hanisch, Ernst, Prof. Dr. Dr., 63110 Rodgau (DE)

(57) **Zusammenfassung**

Es wird die Verwendung eines Fibrinklebers zur Geweberegeneration beschrieben. Im Falle der Behandlung von Leberschädigungen wird dabei durch Injektion eines Fibrinklebers in das hepatobiliäre System oder durch direkte Injektion in das Lebergewebe eine Fibrinkleberocclusion gebildet. Durch die Fibrinkleberocclusion im hepatobiliären System wird die Expression des Hepatozyten-Wachstumsfaktors (HGF) gesteigert und damit die Regeneration des Lebergewebes gefördert. Zusätzlich wird die Verwendung des Fibrinklebers durch Injektion in Lungen-, Pankreas- oder Hautgewebe zur Behandlung des Lungenversagens und des Diabetes mellitus sowie in der plastischen Chirurgie beschrieben.

## Beschreibung

Gegenstand der Erfindung ist eine neue Verwendung eines Fibrinklebers zur Geweberegeneration.

Es ist bekannt, dass in der modernen Chirurgie Fibrinkleber zunehmend an Bedeutung gewinnen, da es sich hierbei um ein gut verträgliches und die Wundheilung förderndes Biomaterial handelt. Die Fibrinklebung wird bisher zur Hämostase von stark blutenden Wunden bei Operationen an parenchymatösen, inneren Organen, bei Hauttransplantationen, in der Notfallchirurgie bei inneren und äußeren Verletzungen, vor allem aber auch zur unterstützenden Abdichtung von Nähten zur Vermeidung postoperativer Blutungen eingesetzt. In der HNO- und Gesichts-Chirurgie wird für die Heilung äußerer Wunden der Fibrinkleber im Nahtverschluss aus kosmetischen Gründen vorgezogen. Außerdem wird der Fibrinkleber zunehmend in der endoskopischen Chirurgie zum Beispiel zur Blutstillung von Magengeschwüren eingesetzt. Die im Handel befindlichen Fibrinkleber wie Beriplast® enthalten als Hauptbestandteile die aus dem Humanplasma gewonnenen Gerinnungsfaktoren Fibrinogen, Thrombin und Faktor XIII.

Es ist außerdem bekannt, dass die Regeneration des durch toxische Einflüsse zerstörten Lebergewebes insbesondere vom Hepatozyten-Wachstumsfaktor (HGF) abhängig ist. Die Produktion des HGFS kann durch ein System unterschiedlicher, aktivierender Proteine gesteigert werden. Am Anfang dieser aktivierenden Kaskade scheint Thrombin eine nicht unwesentliche Rolle zu spielen. Trotzdem ist bisher nicht versucht worden, das im Fibrinkleber enthaltene Thrombin zur Behandlung von Leberschädigungen einzusetzen. Da die Letalität des akuten Leberversagens bis zu 80% beträgt, besteht ein erheblicher Bedarf an prophylaktischen und therapeutischen Maßnahmen, mit denen insbesondere das akute Leberversagen verhindert werden kann.

Beim akuten Leberversagen bestimmen in erster Linie zwei Faktoren das Überleben des Patienten: das Ausmaß der Leberschädigung und die Geschwindigkeit der Hepatozytenregeneration (1). Die Hepatozytenregeneration läßt sich zum Beispiel nach Gallengangsligatur deutlich steigern (2). Dabei zeigen Gallengangszellen nach zwei Tagen eine 17-fache Zunahme der Proliferation, begleitet von einer 4-fachen Steigerung der Hepatozytenregeneration.

Ein Gegenstand der Erfindung ist nun die Verwendung eines Fibrinklebers zur Regeneration des Lebergewebes, bei dem durch Injektion eines Fibrinklebers in das hepatobiliäre System und/oder direkt in das Lebergewebe eine Fibrinkleberocclusion gebildet wird. Diese neue Verwendung des Fibrinklebers stützt sich auf die Beobachtung, dass eine Fibrinkleberocclusion innerhalb von 24 Stunden reversibel ist und zu einer ausgeprägten Proliferation der Gallengangszellen führt. Die damit verbundene Steigerung der Expression des Hepatozyten-Wachstumsfaktors HGF und dessen Rezeptors c-met führt zu einer schnellen Regeneration des Lebergewebes. Der Erfolg der erfindungsgemäßen Verwendung eines Fibrinklebers zur Regeneration des Lebergewebes konnte durch eine hepatobiliäre Fibrinkleberocclusion beim mit Thioacetamid induzierten akuten Leberversagen gezeigt werden. Außerdem können so mit dem Fibrinkleber gemischte additive Faktoren über eine hepatobiliäre Occlusion und/oder durch direkte Injektion in die Leber lokal in der Leber in hoher Dosierung wirksam werden.

Darüber hinaus hat sich nun gezeigt, dass auch die Regeneration anderer Gewebe durch die intraparenchymatöse Gabe von Fibrinkleber signifikant verbessert wird. So wurde in einem Diabetes-Modell der Ratte (Streptozotozin) durch intraduktale/intraparenchymatöse Gabe von Fibrinkleber in das Pankreas die Glukosetoleranz signifikant verbessert. Das zeigt, dass insulinproduzierende Zellen durch die Einwirkung des Fibrinklebers aus Vorstufen aktiviert werden können. Damit bietet sich für die Behandlung von Diabetes mellitus ein völlig neuer Ansatz.

Des weiteren konnte bei einem akuten Lungenversagen der Ratte gezeigt werden, dass durch intrabronchiale und intraparenchymatöse Gabe von Fibrinkleber die Oxygenierung des Organismus signifikant verbessert wird. Damit eröffnet sich die Möglichkeit einer Behandlung des Lungenversagens durch die Verabreichung des Fibrinklebers.

Weiterhin wurde beobachtet, dass die Regenerationsfähigkeit der Haut nach Setzen von Hautläsionen durch die subcutane Gabe von Fibrinklebern erheblich verbessert wird. Zusätzlich wurde dabei die Elastizität der Haut deutlich gesteigert. Damit kann für den Fibrinkleber in der plastischen Chirurgie und der Regeneration der Haut ein neues Einsatzgebiet geschaffen werden.

Mit dem Fibrinkleber vermischte additive Faktoren können dann lokal in hoher Konzentration im Pankreas, in der Lunge oder der Haut wirksam werden.

Zum Nachweis der Wirksamkeit eines Fibrinklebers zur Regeneration des Lebergewebes wurden folgende Versuche und Messungen durchgeführt.

### 1. Versuch

Das Modell des akuten Leberversagens wurde bei Sprague Dawley Ratten mittels Thioacetamid induziert (3). Dabei wurden zwei Gruppen von je sechs Ratten in folgender Weise behandelt: Die Ratten der Gruppe A erhielten an drei aufeinanderfolgenden Tagen Thioacetamid in Dosierungen von 500 mg/kg, 500 mg/kg sowie 250 mg/kg zur gleichen Tageszeit intraperitoneal. Den Tieren der Gruppe B wurden 24 Stunden vor Beginn der Thioacetamidgabe nach Laparotomie in Pentobarbitalnarkose ein langsam aushärtender Fibrinkleber (Beriplast®) in das hepatobiliäre System injiziert.

Folgende Ergebnisse wurden beobachtet: Der histologische Befund der Hepatozyten der Ratten der Gruppe A zeigte eine Kernschwellung mit stark prominenten Nukleolen, darüber hinaus wurde eine ausgedehnte, läppchenzentrale Entparenchymisierung mit lockerer bis dichter rundzelliger Infiltration beobachtet. In der Gruppe B zeigten die Portalfelder ein leichtes Ödem und diskrete Rundzellinfiltration, während sich die Hepatozyten durch eine auffallende Aufhellung mit vereinzelten zentrolobulären Einzelzellnekrosen auszeichneten.

Die Letalität der Gruppe A betrug nach fünf Tagen 100%, während alle Tiere der Gruppe B überlebten. Hieraus ist der Schluss zu ziehen, dass eine hepatobiliäre Fibrinkleberocclusion prophylaktisch den letalen Thioacetamid-induzierten Leberausfall bei der Ratte verhindern kann.

### 2. Versuch

40 SD-Ratten erhielten täglich eine Injektion von 100 mg/kg Thioacetamid intraperitoneal. Am siebenten Tag wurden 20 Tiere unter Äthernarkose laparotomiert, der Gallengang mikrochirurgisch präpariert, kanüliert und nachfolgend 0,2 ml Beriplast®-Fibrinkleber injiziert. 20 Tiere wurden nur laparotomiert (Kontrollgruppe). Anschließend erfolgte die Fortführung der Thioacetamidbehandlung bis zum 14. Tag. Am 14. Tag erfolgte unter Äthernarkose die Laparotomie mit Blutentnahme aus der Aorta und nach erfolgtem Entbluten der Tiere die Organentnahme. Die Bestimmungen der Glutamat-Oxalacetat-Transaminase (GOT), der Glutamat-Pyruvat-Transaminase (GPT), der Gamma-Glutamyl-Transferase (γGT), der alkalischen Phosphatase, des Bilirubins, des Harnstoffs und des Kreatinins erfolgten mit Routinemethoden. Zur immunhistochemischen Aufarbeitung der in Flüssigstickstoff fixierten Lebern wurden diese mit dem Gefriermikrotom in 5 µm dünne Präparate geschnitten und jeweils zwei Präparate auf "Superfrost plus"-Objektträgern aufgezogen. Von jedem Tier wurden sieben Objektträger zur Einfärbung sowie weitere sieben Objektträger als Negativ- bzw. Isotypkontrolle angelegt.

### Verdünnungsstufen:

HGF-alpha: 1:200; HGF-beta: 1:100; c-met: 1:200.

Die Ausgangskonzentration lag jeweils bei 200µg/ml. Die Auswertung immunhistochemischer Färbungen erfolgte ohne Kenntnis der Gruppenzugehörigkeit semiquantitativ mit fünf Abstufungen.

### Ergebnisse:

**Tabelle 1**

| (Daten als Mittelwerte ± 1 SD) | | | | | |
|---|---|---|---|---|---|
| | **GOT** | **GPT** | **γGT** | **AP** | **Bilirubin** |
| Kontrolle | 40 ± 6 | 19 ± 9 | 3 ± 0,9 | 237 ± 42 | 0,7 ± 0,1 |
| Occlusion | 35 ± 8 | 17 ± 5 | 3 ± 0,9 | 335 ± 149 | 0,4 ± 0,1* |

| | | | | | |
|---|---|---|---|---|---|
| *p<0,005 | | | | | |

**Tabelle 2**

| (Daten als Mittelwerte ± 1 SD) | | | |
|---|---|---|---|
| | **c-met** | **HGF-alpha** | **HGF-beta** |
| Kontrolle | 3,33 ± 0,62 | 2,67 ± 1,18 | 0,33 ± 0,47 |
| Occlusion | 3,73 ± 0,44 | 3,73 ± 0,44* | 2,27 ± 1,06* |

| | | | |
|---|---|---|---|
| *p<0,05 | | | |

Diese Versuche zeigen, dass die hepatobiliäre Fibrinkleberocclusion die Expression von HGF in der Thioacetamid-geschädigten Leber steigert. Daraus ergeben sich Anwendungsmöglichkeiten des Fibrinklebers zur Regeneration des Lebergewebes.

### Literaturzusammenstellung:

1. Lake J. und Sussmann N. (1995 Editorial: Determining Prognosis in Patients with Fulminant Hepatic Failure: When You Absolutely, Positively Have to Know the Answer; Hepatology 21, 879-881
2. Polimeno L., Azzarone A., Zeng Q.H., Panella C., Subbotin V., Carr B., Bouzahzah B., Francavilla A., Starzl T.E.(1995), Cell Proliferation and Oncogene Expression after Bile Duct Ligation in the Rat: Evidence of a Specific Growth Effect on Bile Duct Cells; Hepatology 21, 1070-1078
3. Zimmermann Ch., Ferenci P., Pifl Ch., Yurdaydin C., Ebner J., Lassmann H., Roth E., Hörtnagl H. (1989), Hepatic Encephalopathy in Thioacetamide-Induced Acute Liver Failure in Rats: Characterizatian of an Improved Model and Study of Amino Acidergic Neurotransmission; Hepatology 9, 594-601

## Patentansprüche

1. Verwendung eines Fibrinklebers zur Regeneration von Gewebe.

2. Verwendung nach Anspruch 1, **dadurch gekennzeichnet**, dass der Fibrinkleber durch Injektion in die Pankreas eine Aktivierung insulinproduzierender Zellen hervorruft.

3. Verwendung nach Anspruch 1, **dadurch gekennzeichnet**, dass der Fibrinkleber durch Injektion in das intrabronchiale Gewebe die Oxygenierung des Organismus verbessert.

4. Verwendung nach Anspruch 1, **dadurch gekennzeichnet**, dass durch subcutane Injektion des Fibrinklebers die Regenerationsfähigkeit der Haut verbessert wird.

5. Verwendung eines Fibrinklebers zur Regeneration des Lebergewebes.

6. Verwendung nach Anspruch 5, **dadurch gekennzeichnet**, dass durch Injektion eines Fibrinklebers in das hepatobiliäre System eine Fibrinkleberocclusion gebildet wird.

7. Verwendung nach den Ansprüchen 5 und 6, **dadurch gekennzeichnet**, dass durch die Bildung einer Fibrinkleberocclusion im hepatobiliären System die Expression des Hepatozyten-Wachstumsfaktor (HGF) und dessen Rezeptors c-met gesteigert wird.

8. Verwendung nach den Ansprüchen 5 bis 7, **dadurch gekennzeichnet**, dass zusätzlich mit dem Fibrinkleber additive Faktoren vermischt werden und so über eine hepatobiliäre Occlusion lokal in der Leber in hoher Dosierung wirksam werden können.

9. Verwendung nach den Ansprüchen 1 bis 4, **dadurch gekennzeichnet**, dass mit dem Fibrinkleber vermischte additive Faktoren in der Pankreas, in der Haut oder in der Lunge in hoher Dosierung lokal wirksam werden können.
